# EUROPEAN PATENT APPLICATION

(11) **EP 1 484 408 A1**
(43) Date of publication of application: **08.12.2004**
(21) Application number: 04019791.5
(22) Date of filing: 27.09.2000
(51) Int. Cl.: C12N 15/54, C12N 9/12, C07K 16/40, C12N 5/10, A01K 67/027

(54) **Human kinase proteins and polynucleotides encoding the same**

(30) Priority: 28.09.1999 US 156511 P
(62) Divisional of application: 00966996.1
(71) Applicant: Lexicon Genetics Incorporated, The Woodlands, TX 77381-1160 (US)
(72) Inventor: Donoho, Gregory, The Woodlands, TX 77381 (US); Turner, Alexander, The Woodlands, TX 77381 (US); Nehls, Michael, 82131 Stockdorf (DE); Friedrich, Glenn, Houston, TX 7704 (US); Zambrowicz, Brian, The Woodlands, TX 77382 (US); Sands, Arther, The Woodlands, TX 77382 (US)
(74) Representative: Silveston, Judith

(57) **Abstract**

Novel human polynucleotide and polypeptide sequences are disclosed that can be used in therapeutic, diagnostic, and pharmacogenomic applications.

## Description

The present application claims the benefit of U.S. Provisional Application Number 60/156,511 which was filed on September 28, 1999 and is herein incorporated by reference in its entirety.

### 1. INTRODUCTION

The present invention relates to the discovery, identification, and characterization of novel human polynucleotides encoding proteins that share sequence similarity with animal kinases. The invention encompasses the described polynucleotides, host cell expression systems, the encoded proteins, fusion proteins, polypeptides and peptides, antibodies to the encoded proteins and peptides, and genetically engineered animals that either lack or over express the disclosed genes, antagonists and agonists of the proteins, and other compounds that modulate the expression or activity of the proteins encoded by the disclosed genes that can be used for diagnosis, drug screening, clinical trial monitoring and the treatment of physiological disorders.

### 2. BACKGROUND OF THE INVENTION

Kinases mediate phosphorylation of a wide variety of proteins and compounds in the cell. Along with phosphatases, kinases are involved in a wide range of regulatory pathways. Given the physiological importance of kinases, they have been subject to intense scrutiny and are proven drug targets.

### 3. SUMMARY OF THE INVENTION

The present invention relates to the discovery, identification, and characterization of nucleotides that encode novel human proteins, and the corresponding amino acid sequences of these proteins. The novel human proteins (NHPs) described for the first time herein share structural similarity with animal kinases, and more particularly serine/threonine protein kinases. As such, the novel sequences represent a new family of proteins having homologues and orthologs across a range of phyla and species.

The novel human nucleic acid sequences described herein, encode alternative proteins/open reading frames (ORFs) of 187, 356, 324, 198, 347, and 315 amino acids in length (see SEQ ID NOS: 2, 4, 6, 8, 10, and 12).

The invention also encompasses agonists and antagonists of the described NHPs, including small molecules, large molecules, mutant NHPs, or portions thereof that compete with native NHP, peptides, and antibodies, as well as nucleotide sequences that can be used to inhibit the expression of the described NHPs (*e.g*., antisense and ribozyme molecules, and gene or regulatory sequence replacement constructs) or to enhance the expression of the described NHP sequences (*e.g.*, expression constructs that place the described gene under the control of a strong promoter system). The present invention also includes both transgenic animals that express a NHP transgene, and NHP "knock-outs" (which can be conditional) that do not express a functional NHP.

Further, the present invention also relates to processes for identifying compounds that modulate, *i.e.*, act as agonists or antagonists, of NHP expression and/or NHP product activity that utilize purified preparations of the described NHPs and/or NHP product, or cells expressing the same. Such compounds can be used as therapeutic agents for the treatment of any of a wide variety of symptoms associated with biological disorders or imbalances.

### 4. DESCRIPTION OF THE SEQUENCE LISTING AND FIGURES

The Sequence Listing provides the sequence of novel human ORFs that encode the described NHP kinase-like proteins.

### 5. DETAILED DESCRIPTION OF THE INVENTION

The NHPs, described for the first time herein, are novel proteins that are expressed in, *inter alia*, human cell lines, and human brain, pituitary, spinal cord, spleen, trachea, kidney, prostate, testis, and adrenal gland cells. The described sequences were compiled from gene trapped cDNAs, and human testis cDNA libraries, (Clontech, Palo Alto, CA).

The present invention encompasses the nucleotides presented in the Sequence Listing, host cells expressing such nucleotides, the expression products of such nucleotides, and: (a) nucleotides that encode mammalian homologs of the described sequences, including the specifically described NHPs, and the NHP products; (b) nucleotides that encode one or more portions of the NHPs that correspond to functional domains, and the polypeptide products specified by such nucleotide sequences, including but not limited to the novel regions of any active domain(s); (c) isolated nucleotides that encode mutant versions, engineered or naturally occurring, of the described NHPs in which all or a part of at least one domain is deleted or altered, and the polypeptide products specified by such nucleotide sequences, including but not limited to soluble proteins and peptides in which all or a portion of the signal sequence in deleted; (d) nucleotides that encode chimeric fusion proteins containing all or a portion of a coding region of an NHP, or one of its domains (*e.g.*, a receptor/ligand binding domain, accessory protein/self-association domain, etc.) fused to another peptide or polypeptide; or (e) therapeutic or diagnostic derivatives of the described polynucleotides such as oligonucleotides, antisense polynucleotides, ribozymes, dsRNA, or gene therapy constructs comprising a sequence first disclosed in the Sequence Listing.

As discussed above, the present invention includes: (a) the human DNA sequences presented in the Sequence Listing (and vectors comprising the same) and additionally contemplates any nucleotide sequence encoding a contiguous NHP open reading frame (ORF) that hybridizes to a complement of a DNA sequence presented in the Sequence Listing under highly stringent conditions, *e.g.*, hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F.M. *et al*., eds., 1989, Current Protocols in Molecular Biology, Vol. I, Green Publishing Associates, Inc., and John Wiley & sons, Inc., New York, at p. 2.10.3) and encodes a functionally equivalent gene product. Additionally contemplated are any nucleotide sequences that hybridize to the complement of the DNA sequence that encode and express an amino acid sequence presented in the Sequence Listing under moderately stringent conditions, *e.g.,* washing in 0.2xSSC/0.1% SDS at 42°C (Ausubel et al., 1989, *supra*), yet still encode a functionally equivalent NHP product. Functional equivalents of a NHP include naturally occurring NHPs present in other species and mutant NHPs whether naturally occurring or engineered (by site directed mutagenesis, gene shuffling, directed evolution as described in, for example, U.S. Patent No. 5,837,458). The invention also includes degenerate nucleic acid variants of the disclosed NHP polynucleotide sequences.

Additionally contemplated are polynucleotides encoding NHP ORFs, or their functional equivalents, encoded by polynucleotide sequences that are about 99, 95, 90, or about 85 percent similar to corresponding regions of SEQ ID NO:1 (as measured by BLAST sequence comparison analysis using, for example, the GCG sequence analysis package using default parameters).

The invention also includes nucleic acid molecules, preferably DNA molecules, that hybridize to, and are therefore the complements of, the described NHP nucleotide sequences. Such hybridization conditions may be highly stringent or less highly stringent, as described above. In instances where the nucleic acid molecules are deoxyoligonucleotides ("DNA oligos"), such molecules are generally about 16 to about 100 bases long, or about 20 to about 80, or about 34 to about 45 bases long, or any variation or combination of sizes represented therein that incorporate a contiguous region of sequence first disclosed in the Sequence Listing. Such oligonucleotides can be used in conjunction with the polymerase chain reaction (PCR) to screen libraries, isolate clones, and prepare cloning and sequencing templates, etc.

Alternatively, such NHP oligonucleotides can be used as hybridization probes for screening libraries, and assessing gene expression patterns (particularly using a micro array or high-throughput "chip" format). Additionally, a series of the described NHP oligonucleotide sequences, or the complements thereof, can be used to represent all or a portion of the described NHP sequences. The oligonucleotides, typically between about 16 to about 40 (or any whole number within the stated range) nucleotides in length may partially overlap each other and/or the NHP sequence may be represented using oligonucleotides that do not overlap. Accordingly, the described NHP polynucleotide sequences shall typically comprise at least about two or three distinct oligonucleotide sequences of at least about 18, and preferably about 25, nucleotides in length that are each first disclosed in the described Sequence Listing. Such oligonucleotide sequences may begin at any nucleotide present within a sequence in the Sequence Listing and proceed in either a sense (5'-to-3') orientation vis-a-vis the described sequence or in an antisense orientation.

For oligonucleotide probes, highly stringent conditions may refer, *e.g*., to washing in 6xSSC/0.05% sodium pyrophosphate at 37°C (for 14-base oligos), 48°C (for 17-base oligos), 55°C (for 20-base oligos), and 60°C (for 23-base oligos). These nucleic acid molecules may encode or act as NHP sequence antisense molecules, useful, for example, in NHP sequence regulation (for and/or as antisense primers in amplification reactions of NHP gene nucleic acid sequences). With respect to NHP sequence regulation, such techniques can be used to regulate biological functions. Further, such sequences can be used as part of ribozyme and/or triple helix sequences that are also useful for NHP sequence regulation.

Inhibitory antisense or double stranded oligonucleotides can additionally comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide can also comprise at least one modified sugar moiety selected from the group including but not limited to arabinose, 2-fluoroarabinose, xylulose, and hexose.

In yet another embodiment, the antisense oligonucleotide will comprise at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier *et al.,* 1987, Nucl. Acids Res. *15*:6625-6641). The oligonucleotide is a 2'-0-methylribonucleotide (Inoue *et al*., 1987, Nucl. Acids Res. *15*:6131-6148), or a chimeric RNA-DNA analogue (Inoue *et al.,* 1987, FEBS Lett. *215*:327-330). Alternatively, double stranded RNA can be used to disrupt the expression and function of a targeted NHP.

Oligonucleotides of the invention can be synthesized by standard methods known in the art, *e.g.* by use of an automated DNA synthesizer (such as are commercially available from Biosearch, Applied Biosystems, etc.). As examples, phosphorothioate oligonucleotides can be synthesized by the method of Stein *et al.* (1988, Nucl. Acids Res. *16*:3209), and methylphosphonate oligonucleotides can be prepared by use of controlled pore glass polymer supports (Sarin *et al*., 1988, Proc. Natl. Acad. Sci. U.S.A. *85*:7448-7451), etc.

Low stringency conditions are well known to those of skill in the art, and will vary predictably depending on the specific organisms from which the library and the labeled sequences are derived. For guidance regarding such conditions see, for example, Sambrook *et al.,* 1989, Molecular Cloning, A Laboratory Manual (and periodic updates thereof), Cold Springs Harbor Press, N.Y.; and Ausubel *et al*., 1989, Current Protocols in Molecular Biology, Green Publishing Associates and Wiley Interscience, N.Y.

Alternatively, suitably labeled NHP nucleotide probes can be used to screen a human genomic library using appropriately stringent conditions or by PCR. The identification and characterization of human genomic clones is helpful for identifying polymorphisms (including, but not limited to, nucleotide repeats, microsatellite alleles, single nucleotide polymorphisms, or coding single nucleotide polymorphisms), determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (*e.g.*, splice acceptor and/or donor sites), etc., that can be used in diagnostics and pharmacogenomics.

Further, a NHP sequence homolog can be isolated from nucleic acid from an organism of interest by performing PCR using two degenerate or "wobble" oligonucleotide primer pools designed on the basis of amino acid sequences within the NHP products disclosed herein. The template for the reaction may be total RNA, mRNA, and/or cDNA obtained by reverse transcription of mRNA prepared from, for example, human or non-human cell lines or tissue, such as prostate, rectum, colon, or adrenal gland, known or suspected to express an allele of a NHP gene. The PCR product can be subcloned and sequenced to ensure that the amplified sequences represent the sequence of the desired NHP. The PCR fragment can then be used to isolate a full length cDNA clone by a variety of methods. For example, the amplified fragment can be labeled and used to screen a cDNA library, such as a bacteriophage cDNA library. Alternatively, the labeled fragment can be used to isolate genomic clones via the screening of a genomic library.

PCR technology can also be used to isolate full length cDNA sequences. For example, RNA can be isolated, following standard procedures, from an appropriate cellular or tissue source (*i.e*., one known, or suspected, to express a NHP sequence, such as, for example, testis tissue). A reverse transcription (RT) reaction can be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment can be isolated. For a review of cloning strategies that can be used, see *e.g.*, Sambrook *et al.,* 1989, *supra*.

A cDNA encoding a mutant NHP sequence can be isolated, for example, by using PCR. In this case, the first cDNA strand may be synthesized by hybridizing an oligo-dT oligonucleotide to mRNA isolated from tissue known or suspected to be expressed in an individual putatively carrying a mutant NHP allele, and by extending the new strand with reverse transcriptase. The second strand of the cDNA is then synthesized using an oligonucleotide that hybridizes specifically to the 5' end of the normal gene. Using these two primers, the product is then amplified via PCR, optionally cloned into a suitable vector, and subjected to DNA sequence analysis through methods well known to those of skill in the art. By comparing the DNA sequence of the mutant NHP allele to that of a corresponding normal NHP allele, the mutation(s) responsible for the loss or alteration of function of the mutant NHP sequence product can be ascertained.

Alternatively, a genomic library can be constructed using DNA obtained from an individual suspected of or known to carry a mutant NHP allele (*e.g.*, a person manifesting a NHP-associated phenotype such as, for example, immune disorders, obesity, high blood pressure, etc.), or a cDNA library can be constructed using RNA from a tissue known, or suspected, to express a mutant NHP allele. A normal NHP sequence, or any suitable fragment thereof, can then be labeled and used as a probe to identify the corresponding mutant NHP allele in such libraries. Clones containing mutant NHP sequences can then be purified and subjected to sequence analysis according to methods well known to those skilled in the art.

Additionally, an expression library can be constructed utilizing cDNA synthesized from, for example, RNA isolated from a tissue known, or suspected, to express a mutant NHP allele in an individual suspected of or known to carry such a mutant allele. In this manner, gene products made by the putatively mutant tissue may be expressed and screened using standard antibody screening techniques in conjunction with antibodies raised against a normal NHP product, as described below. (For screening techniques, see, for example, Harlow, E. and Lane, eds., 1988, "Antibodies: A Laboratory Manual", Cold Spring Harbor Press, Cold Spring Harbor.)

Additionally, screening can be accomplished by screening with labeled NHP fusion proteins, such as, for example, alkaline phosphatase-NHP or NHP-alkaline phosphatase fusion proteins. In cases where a NHP mutation results in an expressed gene product with altered function (*e.g.*, as a result of a missense or a frameshift mutation), polyclonal antibodies to a NHP are likely to cross-react with a corresponding mutant NHP gene product. Library clones detected via their reaction with such labeled antibodies can be purified and subjected to sequence analysis according to methods well known in the art.

An additional application of the described novel human polynucleotide sequences is their use in the molecular mutagenesis/evolution of proteins that are at least partially encoded by the described novel sequences using, for example, polynucleotide shuffling or related methodologies. Such approaches are described in U.S. Patents Nos. 5,830,721 and 5,837,458 which are herein incorporated by reference in their entirety.

The invention also encompasses (a) DNA vectors that contain any of the foregoing NHP coding sequences and/or their complements (*i.e.*, antisense); (b) DNA expression vectors that contain any of the foregoing NHP coding sequences operatively associated with a regulatory element that directs the expression of the coding sequences (for example, baculo virus as described in U.S. Patent No. 5,869,336 herein incorporated by reference); (c) genetically engineered host cells that contain any of the foregoing NHP coding sequences operatively associated with a regulatory element that directs the expression of the coding sequences in the host cell; and (d) genetically engineered host cells that express an endogenous NHP sequence under the control of an exogenously introduced regulatory element (*i.e*., gene activation). As used herein, regulatory elements include, but are not limited to, inducible and non-inducible promoters, enhancers, operators and other elements known to those skilled in the art that drive and regulate expression. Such regulatory elements include, but are not limited to, the human cytomegalovirus (hCMV) immediate early gene, regulatable, viral elements (particularly retroviral LTR promoters) the early or late promoters of SV40 adenovirus, the *lac* system, the *trp* system, the *TAC* system, the *TRC* system, the major operator and promoter regions of phage lambda, the control regions of fd coat protein, the promoter for 3-phosphoglycerate kinase (PGK), the promoters of acid phosphatase, and the promoters of the yeast α-mating factors.

Where, as in the present instance, some of the described NHP peptides or polypeptides are thought to be cytoplasmic proteins, expression systems can be engineered that produce soluble derivatives of a NHP (corresponding to a NHP extracellular and/or intracellular domains, or truncated polypeptides lacking one or more hydrophobic domains) and/or NHP fusion protein products (especially NHP-Ig fusion proteins, *i.e*., fusions of a NHP domain to an IgFc), NHP antibodies, and anti-idiotypic antibodies (including Fab fragments) that can be used in therapeutic applications. Preferably, the above expression systems are engineered to allow the desired peptide or polypeptide to be recovered from the culture media.

The present invention also encompasses antibodies and anti-idiotypic antibodies (including Fab fragments), antagonists and agonists of the NHP, as well as compounds or nucleotide constructs that inhibit expression of a NHP sequence (transcription factor inhibitors, antisense and ribozyme molecules, or gene or regulatory sequence replacement constructs), or promote the expression of a NHP (*e.g.*, expression constructs in which NHP coding sequences are operatively associated with expression control elements such as promoters, promoter/enhancers, etc.).

The NHPs or NHP peptides, NHP fusion proteins, NHP nucleotide sequences, antibodies, antagonists and agonists can be useful for the detection of mutant NHPs or inappropriately expressed NHPs for the diagnosis of disease. The NHP proteins or peptides, NHP fusion proteins, NHP nucleotide sequences, host cell expression systems, antibodies, antagonists, agonists and genetically engineered cells and animals can be used for screening for drugs (or high throughput screening of combinatorial libraries) effective in the treatment of the symptomatic or phenotypic manifestations of perturbing the normal function of NHP in the body. The use of engineered host cells and/or animals can offer an advantage in that such systems allow not only for the identification of compounds that bind to the endogenous receptor/ligand of a NHP, but can also identify compounds that trigger NHP-mediated activities or pathways.

Finally, the NHP products can be used as therapeutics. For example, soluble derivatives such as NHP peptides/domains corresponding the NHPs, NHP fusion protein products (especially NHP-Ig fusion proteins, *i.e*., fusions of a NHP, or a domain of a NHP, to an IgFc), NHP antibodies and anti-idiotypic antibodies (including Fab fragments), antagonists or agonists (including compounds that modulate or act on downstream targets in a NHP-mediated pathway) can be used to directly treat diseases or disorders. For instance, the administration of an effective amount of soluble NHP, or a NHP-IgFc fusion protein or an anti-idiotypic antibody (or its Fab) that mimics the NHP could activate or effectively antagonize the endogenous NHP or a protein interactive therewith. Nucleotide constructs encoding such NHP products can be used to genetically engineer host cells to express such products *in vivo;* these genetically engineered cells function as "bioreactors" in the body delivering a continuous supply of a NHP, a NHP peptide, or a NHP fusion protein to the body. Nucleotide constructs encoding functional NHPs, mutant NHPs, as well as antisense and ribozyme molecules can also be used in "gene therapy" approaches for the modulation of NHP expression. Thus, the invention also encompasses pharmaceutical formulations and methods for treating biological disorders.

Various aspects of the invention are described in greater detail in the subsections below.

### 5.1 THE NHP SEOUENCES

The cDNA sequences and the corresponding deduced amino acid sequences of the described NHPs are presented in the Sequence Listing. The NHP nucleotide sequences were obtained from human cDNA libraries using probes and/or primers generated from human gene trapped sequence tags.

Expression analysis has provided evidence that the described NHPs can be expressed in human tissues as well as gene trapped human cells. In addition to the serine/threonine kinases, the described NHPs also share significant similarity to a range of additional kinase families such as cell division protein kinases, cyclin dependent kinase, etc. from a range of phyla and species. Given the physiological importance of protein kinases, they have been subject to intense scrutiny as exemplified and discussed in U.S. Patent No. 5,817,479 herein incorporated by reference in its entirety.

During the generation of the described sequences, a polymorphism was identified in the 3' UTR reported in SEQ ID NO:13 (which includes a complete NHP ORF flanked by 5' and 3' sequences.

### 5.2 NHPS AND NHP POLYPEPTIDES

NHPs, polypeptides, peptide fragments, mutated, truncated, or deleted forms of the NHPs, and/or NHP fusion proteins can be prepared for a variety of uses. These uses include, but are not limited to, the generation of antibodies, as reagents in diagnostic assays, for the identification of other cellular gene products related to a NHP, as reagents in assays for screening for compounds that can be as pharmaceutical reagents useful in the therapeutic treatment of mental, biological, or medical disorders and disease.

The Sequence Listing discloses the amino acid sequences encoded by the described NHP sequences. The NHPs have initiator methionines in DNA sequence contexts consistent with eucaryotic translation initiation sites.

The NHP amino acid sequences of the invention include the amino acid sequence presented in the Sequence Listing as well as analogues and derivatives thereof. Further, corresponding NHP homologues from other species are encompassed by the invention. In fact, any NHP protein encoded by the NHP nucleotide sequences described above are within the scope of the invention, as are any novel polynucleotide sequences encoding all or any novel portion of an amino acid sequence presented in the Sequence Listing. The degenerate nature of the genetic code is well known, and, accordingly, each amino acid presented in the Sequence Listing, is generically representative of the well known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Biology", 1986, J. Darnell *et al*. eds., Scientific American Books, New York, NY, herein incorporated by reference) are generically representative of all the various permutations and combinations of nucleic acid sequences that can encode such amino acid sequences.

The invention also encompasses proteins that are functionally equivalent to the NHPs encoded by the presently described nucleotide sequences as judged by any of a number of criteria, including, but not limited to, the ability to bind and cleave a substrate of a NHP, or the ability to effect an identical or complementary downstream pathway, or a change in cellular metabolism (*e.g*., proteolytic activity, ion flux, tyrosine phosphorylation, etc.). Such functionally equivalent NHP proteins include, but are not limited to, additions or substitutions of amino acid residues within the amino acid sequence encoded by the NHP nucleotide sequences described above, but which result in a silent change, thus producing a functionally equivalent gene product. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

A variety of host-expression vector systems can be used to express the NHP nucleotide sequences of the invention. Where the NHP peptide or polypeptide can exist, or has been engineered to exist, as a soluble or secreted molecule, the soluble NHP peptide or polypeptide can be recovered from the culture media. Such expression systems also encompass engineered host cells that express a NHP, or functional equivalent, *in situ*. Purification or enrichment of a NHP from such expression systems can be accomplished using appropriate detergents and lipid micelles and methods well known to those skilled in the art. However, such engineered host cells themselves may be used in situations where it is important not only to retain the structural and functional characteristics of the NHP, but to assess biological activity, *e.g*., in drug screening assays.

The expression systems that may be used for purposes of the invention include but are not limited to microorganisms such as bacteria (*e.g., E. coli, B. subtilis*) transformed with recombinant bacteriophage DNA, plasmid DNA or cosmid DNA expression vectors containing NHP nucleotide sequences; yeast (*e.g., Saccharomyces, Pichia*) transformed with recombinant yeast expression vectors containing NHP nucleotide sequences; insect cell systems infected with recombinant virus expression vectors (*e.g*., baculovirus) containing NHP sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing NHP nucleotide sequences; or mammalian cell systems (*e.g*., COS, CHO, BHK, 293, 3T3) harboring recombinant expression constructs containing promoters derived from the genome of mammalian cells (*e.g.*, metallothionein promoter) or from mammalian viruses (*e.g*., the adenovirus late promoter; the vaccinia virus 7.5K promoter).

In bacterial systems, a number of expression vectors may be advantageously selected depending upon the use intended for the NHP product being expressed. For example, when a large quantity of such a protein is to be produced for the generation of pharmaceutical compositions of or containing NHP, or for raising antibodies to a NHP, vectors that direct the expression of high levels of fusion protein products that are readily purified may be desirable. Such vectors include, but are not limited, to the *E. coli* expression vector pUR278 (Ruther *et al.*, 1983, EMBO J. *2*:1791), in which a NHP coding sequence may be ligated individually into the vector in frame with the *lacZ* coding region so that a fusion protein is produced; pIN vectors (Inouye & Inouye, 1985, Nucleic Acids Res. *13*:3101-3109; Van Heeke & Schuster, 1989, J. Biol. Chem. *264*:5503-5509); and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect system, *Autographa californica* nuclear polyhidrosis virus (AcNPV) is used as a vector to express foreign genes. The virus grows in *Spodoptera frugiperda* cells. A NHP coding sequence may be cloned individually into non-essential regions (for example the polyhedrin gene) of the virus and placed under control of an AcNPV promoter (for example the polyhedrin promoter). Successful insertion of NHP coding sequence will result in inactivation of the polyhedrin gene and production of non-occluded recombinant virus (*i.e.*, virus lacking the proteinaceous coat coded for by the polyhedrin gene). These recombinant viruses are then used to infect Spodoptera frugiperda cells in which the inserted sequence is expressed (*e.g*., see Smith *et al*., 1983, J. Virol. 46: 584; Smith, U.S. Patent No. 4,215,051).

In mammalian host cells, a number of viral-based expression systems may be utilized. In cases where an adenovirus is used as an expression vector, the NHP nucleotide sequence of interest may be ligated to an adenovirus transcription/translation control complex, *e.g.*, the late promoter and tripartite leader sequence. This chimeric sequence may then be inserted in the adenovirus genome by in vitro or in vivo recombination. Insertion in a non-essential region of the viral genome (*e.g*., region E1 or E3) will result in a recombinant virus that is viable and capable of expressing a NHP product in infected hosts (*e.g*., See Logan & Shenk, 1984, Proc. Natl. Acad. Sci. USA *81*:3655-3659). Specific initiation signals may also be required for efficient translation of inserted NHP nucleotide sequences. These signals include the ATG initiation codon and adjacent sequences. In cases where an entire NHP sequence or cDNA, including its own initiation codon and adjacent sequences, is inserted into the appropriate expression vector, no additional translational control signals may be needed. However, in cases where only a portion of a NHP coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bittner *et al*., 1987, Methods in Enzymol. *153*:516-544).

In addition, a host cell strain may be chosen that modulates the expression of the inserted sequences, or modifies and processes the gene product in the specific fashion desired. Such modifications (*e.g.*, glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3, WI38, and in particular, human cell lines.

For long-term, high-yield production of recombinant proteins, stable expression is preferred. For example, cell lines which stably express the NHP sequences described above can be engineered. Rather than using expression vectors which contain viral origins of replication, host cells can be transformed with DNA controlled by appropriate expression control elements (*e.g*., promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the NHP product. Such engineered cell lines may be particularly useful in screening and evaluation of compounds that affect the endogenous activity of the NHP product.

A number of selection systems may be used, including but not limited to the herpes simplex virus thymidine kinase (Wigler, *et al*., 1977, Cell *11*:223), hypoxanthine-guanine phosphoribosyltransferase (Szybalska & Szybalski, 1962, Proc. Natl. Acad. Sci. USA *48*:2026), and adenine phosphoribosyltransferase (Lowy, *et al*., 1980, Cell *22*:817) genes can be employed in tk⁻, hgprt⁻ or aprt⁻ cells, respectively. Also, antimetabolite resistance can be used as the basis of selection for the following genes: dhfr, which confers resistance to methotrexate (Wigler, *et al.,* 1980, Natl. Acad. Sci. USA *77*:3567; O'Hare, *et al.,* 1981, Proc. Natl. Acad. Sci. USA *78*:1527); gpt, which confers resistance to mycophenolic acid (Mulligan & Berg, 1981, Proc. Natl. Acad. Sci. USA *78*:2072); neo, which confers resistance to the aminoglycoside G-418 (Colberre-Garapin, *et al.,* 1981, J. Mol. Biol. *150*:1); and hygro, which confers resistance to hygromycin (Santerre, *et al.,* 1984, Gene *30*:147).

Alternatively, any fusion protein can be readily purified by utilizing an antibody specific for the fusion protein being expressed. For example, a system described by Janknecht *et al*. allows for the ready purification of non-denatured fusion proteins expressed in human cell lines (Janknecht, *et al*., 1991, Proc. Natl. Acad. Sci. USA *88*:8972-8976). In this system, the sequence of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺ nitriloacetic acid-agarose columns and histidine-tagged proteins are selectively eluted with imidazole-containing buffers.

### 5.3 ANTIBODIES TO NHP PRODUCTS

Antibodies that specifically recognize one or more epitopes of a NHP, or epitopes of conserved variants of a NHP, or peptide fragments of a NHP are also encompassed by the invention. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of NHP in a biological sample and may, therefore, be utilized as part of a diagnostic or prognostic technique whereby patients may be tested for abnormal amounts of NHP. Such antibodies may also be utilized in conjunction with, for example, compound screening schemes for the evaluation of the effect of test compounds on expression and/or activity of a NHP sequence product. Additionally, such antibodies can be used in conjunction gene therapy to, for example, evaluate the normal and/or engineered NHP-expressing cells prior to their introduction into the patient. Such antibodies may additionally be used as a method for the inhibition of abnormal NHP activity. Thus, such antibodies may, therefore, be utilized as part of treatment methods.

For the production of antibodies, various host animals may be immunized by injection with the NHP, an NHP peptide (e.g., one corresponding the a functional domain of an NHP), truncated NHP polypeptides (NHP in which one or more domains have been deleted), functional equivalents of the NHP or mutated variant of the NHP. Such host animals may include but are not limited to pigs, rabbits, mice, goats, and rats, to name but a few. Various adjuvants may be used to increase the immunological response, depending on the host species, including but not limited to Freund's adjuvant (complete and incomplete), mineral salts such as aluminum hydroxide or aluminum phosphate, surface active substances such as lysolecithin, pluronic polyols, polyanions, peptides, oil emulsions, and potentially useful human adjuvants such as BCG (bacille Calmette-Guerin) and *Corynebacterium parvum.* Alternatively, the immune response could be enhanced by combination and or coupling with molecules such as keyhole limpet hemocyanin, tetanus toxoid, diptheria toxoid, ovalbumin, cholera toxoid or fragments thereof. Polyclonal antibodies are heterogeneous populations of antibody molecules derived from the sera of the immunized animals.

Monoclonal antibodies, which are homogeneous populations of antibodies to a particular antigen, can be obtained by any technique which provides for the production of antibody molecules by continuous cell lines in culture. These include, but are not limited to, the hybridoma technique of Kohler and Milstein, (1975, Nature *256*:495-497; and U.S. Patent No. 4,376,110), the human B-cell hybridoma technique (Kosbor *et al.,* 1983, Immunology Today *4*:72; Cole *et al.,* 1983, Proc. Natl. Acad. Sci. USA *80*:2026-2030), and the EBV-hybridoma technique (Cole *et al*., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated in vitro or in vivo. Production of high titers of mAbs in vivo makes this the presently preferred method of production.

In addition, techniques developed for the production of "chimeric antibodies" (Morrison *et al*., 1984, Proc. Natl. Acad. Sci., *81*:6851-6855; Neuberger *et al.,* 1984, Nature, *312*:604-608; Takeda *et al*., 1985, Nature, *314*:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. Such technologies are described in U.S. Patents Nos. 6,075,181 and 5,877,397 and their respective disclosures which are herein incorporated by reference in their entirety.

Alternatively, techniques described for the production of single chain antibodies (U.S. Patent 4,946,778; Bird, 1988, Science *242*:423-426; Huston *et al*., 1988, Proc. Natl. Acad. Sci. USA *85*:5879-5883; and Ward *et al*., 1989, Nature *334*:544-546) can be adapted to produce single chain antibodies against NHP sequence products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include, but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse *et al.,* 1989, Science, *246*:1275-1281) to allow rapid and easy identification of monoclonal Fab fragments with the desired specificity.

Antibodies to a NHP can, in turn, be utilized to generate anti-idiotype antibodies that "mimic" a given NHP, using techniques well known to those skilled in the art. (See, *e.g*., Greenspan & Bona, 1993, FASEB J *7(5)*:437-444; and Nissinoff, 1991, J. Immunol. *147(8)*:2429-2438). For example antibodies which bind to a NHP domain and competitively inhibit the binding of NHP to its cognate receptor/ligand can be used to generate anti-idiotypes that "mimic" the NHP and, therefore, bind, activate, or neutralize a NHP, NHP receptor, or NHP ligand. Such anti-idiotypic antibodies or Fab fragments of such anti-idiotypes can be used in therapeutic regimens involving a NHP mediated pathway.

## Claims

1. An isolated nucleic acid molecule comprising at least 24 contiguous bases of nucleotide sequence first disclosed in the NHP polynucleotide sequence described in SEQ ID NO: 9.

2. An isolated nucleic acid molecule comprising a nucleotide sequence that:
(a) encodes the amino acid sequence shown in SEQ ID NO: 10; and
(b) hybridizes under stringent conditions to the nucleotide sequence of SEQ ID NO: 9 or the complement thereof.

3. An isolated nucleic acid molecule comprising a nucleotide sequence encoding the amino acid shown in SEQ ID NO: 12.

4. An isolated nucleic acid molecule comprising at least 24 contiguous bases of nucleotide sequence first disclosed in the NHP polynucleotide described in SEQ ID NO: 3.

5. A polypeptide encoded by a nucleic acid as claimed in any one of claims 1 to 4.

6. A polypeptide comprising at least about 12 amino acids in a sequence first disclosed in SEQ ID NO: 2.

7. An antibody that specifically recognizes a polynucleotide as claimed in claim 5 or claim 6, or an epitope-binding fragment or derivative of said antibody, for example, a polyclonal or monoclonal antibody, a single chain antibody, a Fab fragment, a F(ab')2, a fragment produced by a Fab expression library, or an anti-idiotypic (anti-Id) antibody.

8. A host cell that comprises a nucleic acid as claimed in any one of claims 1 to 4 and is capable of expressing the polypeptide encoded by said nucleic acid, optionally on the surface of the cell.

9. A transgenic animal that is capable of expressing a nucleic acid as claimed in any one of claims 1 to 4, or that is an optionally conditional "knock-out" animal that does not express a functional polypeptide as claimed in claim 5 or claim 6.

10. Use of a nucleic acid as claimed in any one of claims 1 to 4, a polypeptide as claimed claim 5 or claim 6, an antibody as claimed in claim 7, a host cell as claimed in claim 8 or an animal as claimed in claim 9, in a method for screening compounds for the ability to modulate the expression of a nucleic acid as claimed in any one of claims 1 to 4, or a polypeptide as claimed in claims 5 or claim 6, or that modulate the activity of a polypeptide as claimed in claim 5 or claim 6, for example, for high throughput screening of combinatorial libraries.


SEQUENCE LISTING

<110> LEXICON GENETICS INCORPORATED

<120> Novel Human Kinase Proteins and
  Polynucleotides Encoding the Same

<130> 7891(1) EP JSvn/TJH

<150> US 60/156,511
<151> 1999-09-28

<160> 13

<170> FastSEQ for Windows Version 4.0

<210> 1
<211> 561
<212> DNA
<213> homo sapiens

<400> 1
atggaaaagt atgaaaaatt agctaagact ggagaagggt cttatggggt tgtattcaaa     60
tgcagaaaca aaacctctgg acaagtagta gctgttaaaa aatttgtgga atctgaagat    120
gatcctgttg ttaagaaaat agcactaaga gaaatacgta tgttgaagca attaaaacat    180
ccaaatcttg tgaacctcat cgaggtgttc aggagaaaaa ggaaaatgca tttagttttt    240
gaatactgtg atcatacact tttaaatgag ctggaaagaa acccaaatgg agttgctgat    300
ggagtgatca aaagcgtatt atggcaaaca cttcaagctc ttaatttctg tcatatacat    360
aactgtattc acagagatat aaaacctgaa aatattctaa taactaagca aggaataatc    420
aagatttgtg acttcgggtt tgcacaaatt ctgagttgga cttcatcttt ctctggtgcc    480
tccttgattg gcttaatagt tgaccttctg aattcttttt ctgccaattc agagattttt    540
ctcctggctt ggatccattg c                                              561

<210> 2
<211> 187
<212> PRT
<213> homo sapiens

<400> 2
Met Glu Lys Tyr Glu Lys Leu Ala Lys Thr Gly Glu Gly Ser Tyr Gly
 1               5                  10                  15
Val Val Phe Lys Cys Arg Asn Lys Thr Ser Gly Gln Val Val Ala Val
            20                  25                  30
Lys Lys Phe Val Glu Ser Glu Asp Asp Pro Val Val Lys Lys Ile Ala
        35                  40                  45
Leu Arg Glu Ile Arg Met Leu Lys Gln Leu Lys His Pro Asn Leu Val
    50                  55                  60
Asn Leu Ile Glu Val Phe Arg Arg Lys Arg Lys Met His Leu Val Phe
65                  70                  75                  80
Glu Tyr Cys Asp His Thr Leu Leu Asn Glu Leu Glu Arg Asn Pro Asn
                85                  90                  95
Gly Val Ala Asp Gly Val Ile Lys Ser Val Leu Trp Gln Thr Leu Gln
            100                 105                 110
Ala Leu Asn Phe Cys His Ile His Asn Cys Ile His Arg Asp Ile Lys
        115                 120                 125
Pro Glu Asn Ile Leu Ile Thr Lys Gln Gly Ile Ile Lys Ile Cys Asp
    130                 135                 140
Phe Gly Phe Ala Gln Ile Leu Ser Trp Thr Ser Ser Phe Ser Gly Ala
145                 150                 155                 160
Ser Leu Ile Gly Leu Ile Val Asp Leu Leu Asn Ser Phe Ser Ala Asn
                165                 170                 175
Ser Glu Ile Phe Leu Leu Ala Trp Ile His Cys

180                 185

<210> 3
<211> 1068
<212> DNA
<213> homo sapiens

<400> 3
atggaaaagt atgaaaaatt agctaagact ggagaagggt cttatggggt tgtattcaaa      60
tgcagaaaca aaacctctgg acaagtagta gctgttaaaa aatttgtgga atctgaagat     120
gatcctgttg ttaagaaaat agcactaaga gaaatacgta tgttgaagca attaaaacat     180
ccaaatcttg tgaacctcat cgaggtgttc aggagaaaaa ggaaaatgca tttagttttt     240
gaatactgtg atcatacact tttaaatgag ctggaaagaa acccaaatgg agttgctgat     300
ggagtgatca aaagcgtatt atggcaaaca cttcaagctc ttaatttctg tcatatacat     360
aactgtattc acagagatat aaaacctgaa aatattctaa taactaagca aggaataatc     420
aagatttgtg acttcgggtt tgcacaaatt ctgattccag gagatgccta caccgattat     480
gtagctacga gatggtaccg agctcctgaa cttcttgtgg gagatactca gtatggttct     540
tcagtcgata tatgggctat tggttgtgtt tttgcagagc tcctgacagg ccagccactg     600
tggcctggaa aatcagatgt ggaccaactt tatctgataa tcagaacact agtagagacg     660
gggtttcgcc atgttgacca ggctggtctc gaactcttga cgtcaagtga tccacctgcc     720
gtagcctctc aaagtgctgg aattacagga aaattaatcc caagacatca atcaatcttt     780
aaaagtaacg ggtttttcca tggcatcagt atacctgagc cagaagacat ggaaactctt     840
gaggaaaagt tctcagatgt tcatcctgtg gctctgaact tcatgaaggg gtgtctgaag     900
atgaatccag atgacagatt aacctgttcc caactcctgg agagctccta ctttgattct     960
tttcaagagg cccaaattaa aagaaaagca cgtaatgaag gaagaaacag aagacgccaa    1020
caggtcagag gctgtgtttg gctgctgcag ctctgctcca ggctgcat                 1068

<210> 4
<211> 356
<212> PRT
<213> homo sapiens

<400> 4
Met Glu Lys Tyr Glu Lys Leu Ala Lys Thr Gly Glu Gly Ser Tyr Gly
1               5                   10                  15
Val Val Phe Lys Cys Arg Asn Lys Thr Ser Gly Gln Val Val Ala Val
            20                  25                  30
Lys Lys Phe Val Glu Ser Glu Asp Asp Pro Val Val Lys Lys Ile Ala
        35                  40                  45
Leu Arg Glu Ile Arg Met Leu Lys Gln Leu Lys His Pro Asn Leu Val
    50                  55                  60
Asn Leu Ile Glu Val Phe Arg Arg Lys Arg Lys Met His Leu Val Phe
65                  70                  75                  80
Glu Tyr Cys Asp His Thr Leu Leu Asn Glu Leu Glu Arg Asn Pro Asn
                85                  90                  95
Gly Val Ala Asp Gly Val Ile Lys Ser Val Leu Trp Gln Thr Leu Gln
            100                 105                 110
Ala Leu Asn Phe Cys His Ile His Asn Cys Ile His Arg Asp Ile Lys
        115                 120                 125
Pro Glu Asn Ile Leu Ile Thr Lys Gln Gly Ile Ile Lys Ile Cys Asp
    130                 135                 140
Phe Gly Phe Ala Gln Ile Leu Ile Pro Gly Asp Ala Tyr Thr Asp Tyr
145                 150                 155                 160
Val Ala Thr Arg Trp Tyr Arg Ala Pro Glu Leu Leu Val Gly Asp Thr
                165                 170                 175
Gln Tyr Gly Ser Ser Val Asp Ile Trp Ala Ile Gly Cys Val Phe Ala
            180                 185                 190
Glu Leu Leu Thr Gly Gln Pro Leu Trp Pro Gly Lys Ser Asp Val Asp
        195                 200                 205
Gln Leu Tyr Leu Ile Ile Arg Thr Leu Val Glu Thr Gly Phe Arg His
    210                 215                 220
Val Asp Gln Ala Gly Leu Glu Leu Leu Thr Ser Ser Asp Pro Pro Ala
225                 230                 235                 240

Val Ala Ser Gln Ser Ala Gly Ile Thr Gly Lys Leu Ile Pro Arg His
                245                 250                 255
Gln Ser Ile Phe Lys Ser Asn Gly Phe Phe His Gly Ile Ser Ile Pro
            260                 265                 270
Glu Pro Glu Asp Met Glu Thr Leu Glu Glu Lys Phe Ser Asp Val His
        275                 280                 285
Pro Val Ala Leu Asn Phe Met Lys Gly Cys Leu Lys Met Asn Pro Asp
    290                 295                 300
Asp Arg Leu Thr Cys Ser Gln Leu Leu Glu Ser Ser Tyr Phe Asp Ser
305                 310                 315                 320
Phe Gln Glu Ala Gln Ile Lys Arg Lys Ala Arg Asn Glu Gly Arg Asn
                325                 330                 335
Arg Arg Arg Gln Gln Val Arg Gly Cys Val Trp Leu Leu Gln Leu Cys
            340                 345                 350
Ser Arg Leu His
        355

<210> 5
<211> 972
<212> DNA
<213> homo sapiens

<400> 5
atggaaaagt atgaaaaatt agctaagact ggagaagggt cttatggggt tgtattcaaa      60
tgcagaaaca aaacctctgg acaagtagta gctgttaaaa aatttgtgga atctgaagat     120
gatcctgttg ttaagaaaat agcactaaga gaaatacgta tgttgaagca attaaaacat     180
ccaaatcttg tgaacctcat cgaggtgttc aggagaaaaa ggaaaatgca tttagttttt     240
gaatactgtg atcatacact tttaaatgag ctggaaagaa acccaaatgg agttgctgat     300
ggagtgatca aaagcgtatt atggcaaaca cttcaagctc ttaatttctg tcatatacat     360
aactgtattc acagagatat aaaacctgaa aatattctaa taactaagca aggaataatc     420
aagatttgtg acttcgggtt tgcacaaatt ctgattccag gagatgccta caccgattat     480
gtagctacga gatggtaccg agctcctgaa cttcttgtgg gagatactca gtatggttct     540
tcagtcgata tatgggctat tggttgtgtt tttgcagagc tcctgacagg ccagccactg     600
tggcctggaa aatcagatgt ggaccaactt tatctgataa tcagaacact aggaaaatta     660
atcccaagac atcaatcaat ctttaaaagt aacgggtttt tccatggcat cagtatacct     720
gagccagaag acatggaaac tcttgaggaa aagttctcag atgttcatcc tgtggctctg     780
aacttcatga aggggtgtct gaagatgaat ccagatgaca gattaacctg ttcccaactc     840
ctggagagct cctactttga ttcttttcaa gaggcccaaa ttaaaagaaa agcacgtaat     900
gaaggaagaa acagaagacg ccaacaggtc agaggctgtg tttggctgct gcagctctgc     960
tccaggctgc at                                                         972

<210> 6
<211> 324
<212> PRT
<213> homo sapiens

<400> 6
Met Glu Lys Tyr Glu Lys Leu Ala Lys Thr Gly Glu Gly Ser Tyr Gly
1               5                   10                  15
Val Val Phe Lys Cys Arg Asn Lys Thr Ser Gly Gln Val Val Ala Val
            20                  25                  30
Lys Lys Phe Val Glu Ser Glu Asp Asp Pro Val Val Lys Lys Ile Ala
        35                  40                  45
Leu Arg Glu Ile Arg Met Leu Lys Gln Leu Lys His Pro Asn Leu Val
    50                  55                  60
Asn Leu Ile Glu Val Phe Arg Arg Lys Arg Lys Met His Leu Val Phe
65                  70                  75                  80
Glu Tyr Cys Asp His Thr Leu Leu Asn Glu Leu Glu Arg Asn Pro Asn
                85                  90                  95
Gly Val Ala Asp Gly Val Ile Lys Ser Val Leu Trp Gln Thr Leu Gln
            100                 105                 110
Ala Leu Asn Phe Cys His Ile His Asn Cys Ile His Arg Asp Ile Lys
        115                 120                 125

Pro Glu Asn Ile Leu Ile Thr Lys Gln Gly Ile Ile Lys Ile Cys Asp
    130                 135                 140
Phe Gly Phe Ala Gln Ile Leu Ile Pro Gly Asp Ala Tyr Thr Asp Tyr
145                 150                 155                 160
Val Ala Thr Arg Trp Tyr Arg Ala Pro Glu Leu Leu Val Gly Asp Thr
                165                 170                 175
Gln Tyr Gly Ser Ser Val Asp Ile Trp Ala Ile Gly Cys Val Phe Ala
            180                 185                 190
Glu Leu Leu Thr Gly Gln Pro Leu Trp Pro Gly Lys Ser Asp Val Asp
        195                 200                 205
Gln Leu Tyr Leu Ile Ile Arg Thr Leu Gly Lys Leu Ile Pro Arg His
    210                 215                 220
Gln Ser Ile Phe Lys Ser Asn Gly Phe Phe His Gly Ile Ser Ile Pro
225                 230                 235                 240
Glu Pro Glu Asp Met Glu Thr Leu Glu Glu Lys Phe Ser Asp Val His
                245                 250                 255
Pro Val Ala Leu Asn Phe Met Lys Gly Cys Leu Lys Met Asn Pro Asp
            260                 265                 270
Asp Arg Leu Thr Cys Ser Gln Leu Leu Glu Ser Ser Tyr Phe Asp Ser
        275                 280                 285
Phe Gln Glu Ala Gln Ile Lys Arg Lys Ala Arg Asn Glu Gly Arg Asn
    290                 295                 300
Arg Arg Arg Gln Gln Val Arg Gly Cys Val Trp Leu Leu Gln Leu Cys
305                 310                 315                 320
Ser Arg Leu His

<210> 7
<211> 594
<212> DNA
<213> homo sapiens

<400> 7
atggaaaagt atgaaaaatt agctaagact ggagaagggt cttatggggt tgtattcaaa      60
tgcagaaaca aaacctctgg acaagtagta gctgttaaaa aatttgtgga atctgaagat     120
gatcctgttg ttaagaaaat agcactaaga gaaatacgta tgttgaagca attaaaacat     180
ccaaatcttg tgaacctcat cgaggtgttc aggagaaaaa ggaaaatgca tttagttttt     240
gaatactgtg atcatacact tttaaatgag ctggaaagaa acccaaatgg agttgctgat     300
ggagtgatca aaagcgtatt atggcaaaca cttcaagctc ttaatttctg tcatatacat     360
aactgtattc acagagatat aaaacctgaa aatattctaa taactaagca aggaataatc     420
aagatttgtg acttcgggtt tgcacaaatt ctgagttgga cttcatcttt ctctggtgcc     480
tccttgattg gcttaatagt tgaccttctg aattcttttt ctgccaattc agagattttt     540
ctcctggctt ggatccattg ctggaaaatt aatcccaaga catcaatcaa tctt           594

<210> 8
<211> 198
<212> PRT
<213> homo sapiens

<400> 8
Met Glu Lys Tyr Glu Lys Leu Ala Lys Thr Gly Glu Gly Ser Tyr Gly
 1               5                  10                  15
Val Val Phe Lys Cys Arg Asn Lys Thr Ser Gly Gln Val Val Ala Val
            20                  25                  30
Lys Lys Phe Val Glu Ser Glu Asp Asp Pro Val Val Lys Lys Ile Ala
        35                  40                  45
Leu Arg Glu Ile Arg Met Leu Lys Gln Leu Lys His Pro Asn Leu Val
    50                  55                  60
Asn Leu Ile Glu Val Phe Arg Arg Lys Arg Lys Met His Leu Val Phe
65                  70                  75                  80
Glu Tyr Cys Asp His Thr Leu Leu Asn Glu Leu Glu Arg Asn Pro Asn
                85                  90                  95
Gly Val Ala Asp Gly Val Ile Lys Ser Val Leu Trp Gln Thr Leu Gln

                100                 105                 110
Ala Leu Asn Phe Cys His Ile His Asn Cys Ile His Arg Asp Ile Lys
        115                 120                 125
Pro Glu Asn Ile Leu Ile Thr Lys Gln Gly Ile Ile Lys Ile Cys Asp
    130                 135                 140
Phe Gly Phe Ala Gln Ile Leu Ser Trp Thr Ser Ser Phe Ser Gly Ala
145                 150                 155                 160
Ser Leu Ile Gly Leu Ile Val Asp Leu Leu Asn Ser Phe Ser Ala Asn
                165                 170                 175
Ser Glu Ile Phe Leu Leu Ala Trp Ile His Cys Trp Lys Ile Asn Pro
            180                 185                 190
Lys Thr Ser Ile Asn Leu
        195

<210> 9
<211> 1041
<212> DNA
<213> homo sapiens

<400> 9
atggaaaagt atgaaaaatt agctaagact ggagaagggt cttatggggt tgtattcaaa      60
tgcagaaaca aaacctctgg acaagtagta gctgttaaaa aatttgtgga atctgaagat     120
gatcctgttg ttaagaaaat agcactaaga gaaatacgta tgttgaagca attaaaacat     180
ccaaatcttg tgaacctcat cgaggtgttc aggagaaaaa ggaaaatgca tttagttttt     240
gaatactgtg atcatacact tttaaatgag ctggaaagaa acccaaatgg agttgctgat     300
ggagtgatca aaagcgtatt atggcaaaca cttcaagctc ttaatttctg tcatatacat     360
aactgtattc acagagatat aaaacctgaa aatattctaa taactaagca aggaataatc     420
aagatttgtg acttcgggtt tgcacaaatt ctgattccag gagatgccta caccgattat     480
gtagctacga gatggtaccg agctcctgaa cttcttgtgg gagatactca gtatggttct     540
tcagtcgata tatgggctat tggttgtgtt tttgcagagc tcctgacagg ccagccactg     600
tggcctggaa aatcagatgt ggaccaactt tatctgataa tcagaacact agtagagacg     660
gggtttcgcc atgttgacca ggctggtctc gaactcttga cgtcaagtga tccacctgcc     720
gtagcctctc aaagtgctgg aattacagga aaattaatcc caagacatca atcaatcttt     780
aaaagtaacg ggtttttcca tggcatcagt atacctgagc cagaagacat ggaaactctt     840
gaggaaaagt tctcagatgt tcatcctgtg gctctgaact tcatgaaggg gtgtctgaag     900
atgaatccag atgacagatt aacctgttcc caactcctgg agagctccta ctttgattct     960
tttcaagagg cccaaattaa aagaaaagca cgtaatgaag gaagaaacag aagacgccaa    1020
caggtacttc cgctcaaaag t                                              1041

<210> 10
<211> 347
<212> PRT
<213> homo sapiens

<400> 10
Met Glu Lys Tyr Glu Lys Leu Ala Lys Thr Gly Glu Gly Ser Tyr Gly
1               5                   10                  15
Val Val Phe Lys Cys Arg Asn Lys Thr Ser Gly Gln Val Val Ala Val
            20                  25                  30
Lys Lys Phe Val Glu Ser Glu Asp Asp Pro Val Val Lys Lys Ile Ala
        35                  40                  45
Leu Arg Glu Ile Arg Met Leu Lys Gln Leu Lys His Pro Asn Leu Val
    50                  55                  60
Asn Leu Ile Glu Val Phe Arg Arg Lys Arg Lys Met His Leu Val Phe
65                  70                  75                  80
Glu Tyr Cys Asp His Thr Leu Leu Asn Glu Leu Glu Arg Asn Pro Asn
                85                  90                  95
Gly Val Ala Asp Gly Val Ile Lys Ser Val Leu Trp Gln Thr Leu Gln
            100                 105                 110
Ala Leu Asn Phe Cys His Ile His Asn Cys Ile His Arg Asp Ile Lys
        115                 120                 125
Pro Glu Asn Ile Leu Ile Thr Lys Gln Gly Ile Ile Lys Ile Cys Asp
    130                 135                 140

Phe Gly Phe Ala Gln Ile Leu Ile Pro Gly Asp Ala Tyr Thr Asp Tyr
145                 150                 155                 160
Val Ala Thr Arg Trp Tyr Arg Ala Pro Glu Leu Leu Val Gly Asp Thr
                165                 170                 175
Gln Tyr Gly Ser Ser Val Asp Ile Trp Ala Ile Gly Cys Val Phe Ala
            180                 185                 190
Glu Leu Leu Thr Gly Gln Pro Leu Trp Pro Gly Lys Ser Asp Val Asp
        195                 200                 205
Gln Leu Tyr Leu Ile Ile Arg Thr Leu Val Glu Thr Gly Phe Arg His
    210                 215                 220
Val Asp Gln Ala Gly Leu Glu Leu Leu Thr Ser Ser Asp Pro Pro Ala
225                 230                 235                 240
Val Ala Ser Gln Ser Ala Gly Ile Thr Gly Lys Leu Ile Pro Arg His
                245                 250                 255
Gln Ser Ile Phe Lys Ser Asn Gly Phe Phe His Gly Ile Ser Ile Pro
            260                 265                 270
Glu Pro Glu Asp Met Glu Thr Leu Glu Glu Lys Phe Ser Asp Val His
        275                 280                 285
Pro Val Ala Leu Asn Phe Met Lys Gly Cys Leu Lys Met Asn Pro Asp
    290                 295                 300
Asp Arg Leu Thr Cys Ser Gln Leu Leu Glu Ser Ser Tyr Phe Asp Ser
305                 310                 315                 320
Phe Gln Glu Ala Gln Ile Lys Arg Lys Ala Arg Asn Glu Gly Arg Asn
                325                 330                 335
Arg Arg Arg Gln Gln Val Leu Pro Leu Lys Ser
            340                 345

<210> 11
<211> 945
<212> DNA
<213> homo sapiens

<400> 11
atggaaaagt atgaaaaatt agctaagact ggagaagggt cttatggggt tgtattcaaa      60
tgcagaaaca aaacctctgg acaagtagta gctgttaaaa aatttgtgga atctgaagat     120
gatcctgttg ttaagaaaat agcactaaga gaaatacgta tgttgaagca attaaaacat     180
ccaaatcttg tgaacctcat cgaggtgttc aggagaaaaa ggaaaatgca tttagttttt     240
gaatactgtg atcatacact tttaaatgag ctggaaagaa acccaaatgg agttgctgat     300
ggagtgatca aaagcgtatt atggcaaaca cttcaagctc ttaatttctg tcatatacat     360
aactgtattc acagagatat aaaacctgaa aatattctaa taactaagca aggaataatc     420
aagatttgtg acttcgggtt tgcacaaatt ctgattccag gagatgccta caccgattat     480
gtagctacga gatggtaccg agctcctgaa cttcttgtgg gagatactca gtatggttct     540
tcagtcgata tatgggctat tggttgtgtt tttgcagagc tcctgacagg ccagccactg     600
tggcctggaa aatcagatgt ggaccaactt tatctgataa tcagaacact aggaaaatta     660
atcccaagac atcaatcaat ctttaaaagt aacgggtttt tccatggcat cagtatacct     720
gagccagaag acatggaaac tcttgaggaa aagttctcag atgttcatcc tgtggctctg     780
aacttcatga aggggtgtct gaagatgaat ccagatgaca gattaacctg ttcccaactc     840
ctggagagct cctactttga ttcttttcaa gaggcccaaa ttaaaagaaa agcacgtaat     900
gaaggaagaa acagaagacg ccaacaggta cttccgctca aaagt                     945

<210> 12
<211> 315
<212> PRT
<213> homo sapiens

<400> 12
Met Glu Lys Tyr Glu Lys Leu Ala Lys Thr Gly Glu Gly Ser Tyr Gly
1               5                   10                  15
Val Val Phe Lys Cys Arg Asn Lys Thr Ser Gly Gln Val Val Ala Val
            20                  25                  30
Lys Lys Phe Val Glu Ser Glu Asp Asp Pro Val Val Lys Lys Ile Ala
        35                  40                  45
Leu Arg Glu Ile Arg Met Leu Lys Gln Leu Lys His Pro Asn Leu Val

50                  55                  60
Asn Leu Ile Glu Val Phe Arg Arg Lys Arg Lys Met His Leu Val Phe
65                  70                  75                  80
Glu Tyr Cys Asp His Thr Leu Leu Asn Glu Leu Glu Arg Asn Pro Asn
                85                  90                  95
Gly Val Ala Asp Gly Val Ile Lys Ser Val Leu Trp Gln Thr Leu Gln
            100                 105                 110
Ala Leu Asn Phe Cys His Ile His Asn Cys Ile His Arg Asp Ile Lys
        115                 120                 125
Pro Glu Asn Ile Leu Ile Thr Lys Gln Gly Ile Ile Lys Ile Cys Asp
    130                 135                 140
Phe Gly Phe Ala Gln Ile Leu Ile Pro Gly Asp Ala Tyr Thr Asp Tyr
145                 150                 155                 160
Val Ala Thr Arg Trp Tyr Arg Ala Pro Glu Leu Leu Val Gly Asp Thr
                165                 170                 175
Gln Tyr Gly Ser Ser Val Asp Ile Trp Ala Ile Gly Cys Val Phe Ala
            180                 185                 190
Glu Leu Leu Thr Gly Gln Pro Leu Trp Pro Gly Lys Ser Asp Val Asp
        195                 200                 205
Gln Leu Tyr Leu Ile Ile Arg Thr Leu Gly Lys Leu Ile Pro Arg His
    210                 215                 220
Gln Ser Ile Phe Lys Ser Asn Gly Phe Phe His Gly Ile Ser Ile Pro
225                 230                 235                 240
Glu Pro Glu Asp Met Glu Thr Leu Glu Glu Lys Phe Ser Asp Val His
                245                 250                 255
Pro Val Ala Leu Asn Phe Met Lys Gly Cys Leu Lys Met Asn Pro Asp
            260                 265                 270
Asp Arg Leu Thr Cys Ser Gln Leu Leu Glu Ser Ser Tyr Phe Asp Ser
        275                 280                 285
Phe Gln Glu Ala Gln Ile Lys Arg Lys Ala Arg Asn Glu Gly Arg Asn
    290                 295                 300
Arg Arg Arg Gln Gln Val Leu Pro Leu Lys Ser
305                 310                 315

<210> 13
<211> 1819
<212> DNA
<213> homo sapiens

<400> 13
   ctccgagcga cacgcgcggg agctggggct ggggctgttc ggcgctgctc gaagcttcgt      60
   caccgtcgcc ctgtgggtgc agtgcagcat tgtactgcaa gtcaatcgat acaataattt     120
   aagtcacttc agctataatg gaaaagtatg aaaaattagc taagactgga gaagggtctt     180
   atggggttgt attcaaatgc agaaacaaaa cctctggaca agtagtagct gttaaaaaat     240
   ttgtggaatc tgaagatgat cctgttgtta agaaaatagc actaagagaa atacgtatgt     300
   tgaagcaatt aaaacatcca aatcttgtga acctcatcga ggtgttcagg agaaaaagga     360
   aaatgcattt agtttttgaa tactgtgatc atacactttt aaatgagctg gaaagaaacc     420
   caaatggagt tgctgatgga gtgatcaaaa gcgtattatg gcaaacactt caagctctta     480
   atttctgtca tatacataac tgtattcaca gagatataaa acctgaaaat attctaataa     540
   ctaagcaagg aataatcaag atttgtgact tcgggtttgc acaaattctg agttggactt     600
   catctttctc tggtgcctcc ttgattggct taatagttga ccttctgaat tctttttctg     660
   ccaattcaga gatttttctc ctggcttgga tccattgctg acacagtgtt tcaccatggg     720
   gcccaggctc atctcgaact tctggcctca agtgatcctt ccacctcggc ctcccaaagt     780
   gctggattgc aagtgtgagc caccgtgccc agccagattt ttcaaacaat aactactgag     840
   agctcacaag attgttttta gtgggaacac aatttcgaac aaattcttga gaacgcattc     900
   caggagatgc ctacaccgat tatgtagcta cgagatggta ccgagctcct gaacttcttg     960
   tgggagatac tcagtatggt tcttcagtcg atatatgggc tattggttgt gtttttgcag    1020
   agctcctgac aggccagcca ctgtggcctg gaaaatcaga tgtggaccaa ctttatctga    1080
   taatcagaac actagtagag acggggtttc gccatgttga ccaggctggt ctcgaactct    1140
   tgacgtcaag tgatccacct gccgtagcct ctcaaagtgc tggaattaca ggaaaattaa    1200
   tcccaagaca tcaatcaatc tttaaaagta acgggttttt ccatggcatc agtatacctg    1260
   agccagaaga catggaaact cttgaggaaa agttctcaga tgttcatcct gtggctctga    1320
   acttcatgaa ggggtgtctg aagatgaatc cagatgacag attaacctgt tcccaactcc    1380
   tggagagctc ctactttgat tcttttcaag aggcccaaat taaaagaaaa gcacgtaatg 1440
   aaggaagaaa cagaagacgc caacaggtca gaggctgtgt ttggctgctg cagctctgct 1500
   ccaggctgca ttgagaatcg atttcgagtg tcttctcatt cagggaccca gccaaggagc 1560
   agctcatatg ggaaatatgc ccttctcatg gcagaggccc gaacttgaat acctaatgtc 1620
   tgtcaggagt gacttccgct caaaagttaa agtgctataa aaataattcc ttttttgttt 1680
   ttgrttggct gcctttcaaa gtgagacaag gtggacacca agacctttca tttgtactgg 1740
   tgtagtgatt gctagcttaa taaatattgg gaattgatgt ataaaaccct agactatgaa 1800
   aatatcaaaa aaaaaaaaa 1819